(19) Europäisches Patentamt
European Patent Office
Office européen des brevets

(11) Veröffentlichungsnummer: **0 226 074**
**A1**

(12) # EUROPÄISCHE PATENTANMELDUNG

(21) Anmeldenummer: 86116245.1

(22) Anmeldetag: 24.11.86

(51) Int. Cl.4: C07D 231/40 , C07D 401/04 , A01N 43/56

(30) Priorität: 05.12.85 DE 3543035

(43) Veröffentlichungstag der Anmeldung: 24.06.87 Patentblatt 87/26

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Gehring, Reinhold, Dr.
Dasnoeckel 49
D-5600 Wuppertal 1(DE)
Erfinder: Schallner, Otto, Dr.
Noldeweg 22
D-4019 Monheim(DE)
Erfinder: Stetter, Jörg, Dr.
Gellertweg 4
D-5600 Wuppertal 1(DE)
Erfinder: Santel, Hans-Joachim, Dr.
Grünstrasse 9a
D-5090 Leverkusen(DE)
Erfinder: Schmidt, Robert R., Dr.
Im Waldwinkel 110
D-5060 Bergisch-Gladbach 2(DE)
Erfinder: Lürssen, Klaus, Dr.
August-Kierspel-Strasse 151
D-5060 Bergisch-Gladbach 2(DE)

(54) **5-Fluoracylamino-4-nitro-1-aryl-pyrazole.**

(57) Die Erfindung betrifft 5-Fluoracylamino-4-nitro-1-aryl-pyrazole der Formel (I),

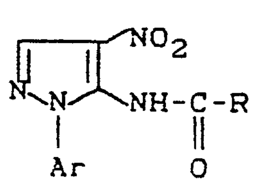

(I)

in welcher
R für durch Fluor substituiertes Alkyl steht und
Ar für jeweils gegebenenfalls substituiertes Phenyl oder Pyridyl steht,
mehrere Verfahren zu ihrer Herstellung und ihre Verwendung als Herbizide und/oder Wachstumsregulatoren.

EP 0 226 074 A1

Xerox Copy Centre

## 5-Fluoracylamino-4-nitro-1-aryl-pyrazole

Die Erfindung betrifft neue 5-Fluoracylamino-4-nitro-1-aryl-pyrazole, mehrere Verfahren zu ihrer Herstellung und ihre Verwendung als Herbizide und Wachstumsregulatoren.

Es ist bereits bekannt, daß bestimmte 5-Halogenacylamino-4-nitro-1-aryl-pyrazole, wie beispielsweise das 5-(ω-Chlorbutyramido)-4-nitro-1-(2,6-dichlor-4-trifluormethylphenyl)-pyrazol herbizide Eigenschaften besitzen (vgl. DE-OS 34 02 308).

Die herbizide Wirksamkeit dieser vorbekannten Verbindungen gegenüber Problemunkräutern ist jedoch, ebenso wie ihre Verträglichkeit gegenüber wichtigen Nutzpflanzen nicht immer in allen Anwendungsbereichen völlig zufriedenstellend.

Es wurden neue 5-Fluoracylamino-4-nitro-1-aryl-pyrazole der allgemeinen Formel (I),

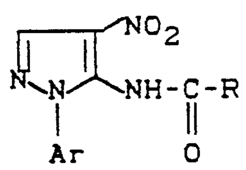

(I)

in welcher
R für durch Fluor substituiertes Alkyl steht und
Ar für jeweils gegebenenfalls substituiertes Phenyl oder Pyridyl steht,
gefunden.

Weiterhin wurde gefunden, daß man die neuen 5-Fluoracylamino-4-nitro-1-aryl-pyrazole der allgemeinen Formel (I),

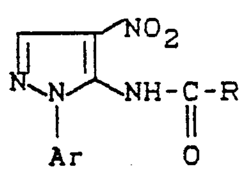

(I)

in welcher
R für durch Fluor substituiertes Alkyl steht und
Ar für jeweils gegebenenfalls substituiertes Phenyl oder Pyridyl steht,
erhält, wenn man

(a) 5-Amino-4-nitro-1-aryl-pyrazole der Formel (II),

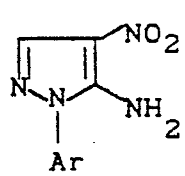

(II)

in welcher
AR die oben angegebene Bedeutung hat, mit Fluoracyl-Verbindungen der Formel (III),

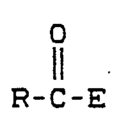

(III)

in welcher
R die oben angegebene Bedeutung hat und
E für eine elektronenanziehende Abgangsgruppe steht,
gegebenenfalls in Gegenwart eines Verdünnungsmittels und gegebenenfalls in Gegenwart eines Säurebindemittels umsetzt, oder wenn man

(b) in 4-Stellung unsubstituierte 5-Fluoracylamino-1-aryl-pyrazole der Formel (IV),

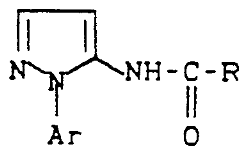

(IV)

in welcher
R die oben angegebene Bedeutung hat, mit Salpetersäure gegebenenfalls in Gegenwart eines Verdünnungsmittels und gegebenenfalls in Gegenwart eines Katalysators umsetzt.

Schließlich wurde gefunden, daß die neuen 5-Fluoracylamino-4-nitro-1-aryl-pyrazole der Formel (I) herbizide und wachstumsregulierende Wirkung haben.

Überraschenderweise zeigen die erfindungsgemäßen 5-Fluoracylamino-4-nitro-1-aryl-pyrazole der allgemeinen Formel (I) eine erheblich bessere herbizide Wirksamkeit gegenüber Problemunkräutern bei vergleichbar guter Nutzpflanzenselektivität als die aus dem Stand der Technik bekannten 5-Halogenacylamino-4-nitro-1-aryl-pyrazole, wie beispielsweise das 5-(ω-Chlorbutyramido)-4-nitro-1-(2,6-dichlor-4-trifluormethyl-phenyl)-pyrazol, welches chemisch und wirkungsmäßig naheliegende Verbindungen sind.

Die erfindungsgemäßen 5-Fluoracylamino-4-nitro-1-aryl-pyrazole sind durch die Formel (I) allgemein definiert. Bevorzugt sind Verbindungen der Formel (I), bei welchen

R für einen Rest 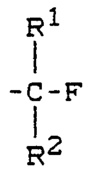 oder für einen Rest

$-A-CF_3$ steht und
Ar für gegebenenfalls einfach oder mehrfach, gleich oder verschieden substituiertes Phenyl oder für jeweils gegebenenfalls einfach oder mehrfach, gleich oder verschieden substituiertes 2-Pyridyl, 3-Pyridyl oder 4-Pyridyl steht, wobei als Substituenten jeweils in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_m-R^3$,
wobei
$R^1$ für Wasserstoff, Fluor, Chlor, geradkettiges oder verzweigtes Halogenalkyl mit 1 bis 4 Kohlenstoffatomen und 1 bis 9 gleichen oder verschiedenen Halogenatomen, oder für jeweils geradkettiges oder verzweigtes Alkyl, Alkoxy oder Alkylthio mit jeweils 1 bis 4 Kohlenstoffatomen steht,
$R^2$ für Wasserstoff, Fluor oder Chlor steht,
$R^3$ für Amino sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,
A für gegebenenfalls einfach oder mehrfach durch Chlor substituiertes Alkylen mit 1 bis 12 Kohlenstoffatomen steht und
m für eine Zahl 0, 1 oder 2 steht.

Besonders bevorzugt sind Verbindungen der Formel (I), bei welchen

R für einen Rest 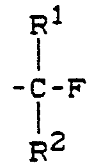 oder für einen Rest $-A-CF_3$ steht

und

Ar für gegebenenfalls ein-bis fünffach, gleich oder verschieden substituiertes Phenyl oder für jeweils gegebenenfalls ein-bis vierfach, gleich oder verschieden substituiertes 2-Pyridyl, 3-Pyridyl oder 4-Pyridyl steht, wobei als Substituenten jeweils in Frage kommen: Cyano, Nitro, Fluor, Chlor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluroethoxy, Trifluorchlorethoxy, Trifluorethoxy, Difluordichlorethoxy,Trifluordichlorethoxy, Pentachlorethoxy oder ein Rest $-S(O)_m-R^3$, wobei

$R^1$ für Wasserstoff, Fluor, Chlor, Trifluormethyl, Difluormethyl, Fluormethyl, Dichlorfluormethyl, Difluorchlormethyl, Chlormethyl, Dichlormethyl, Trichlormethyl, Pentafluorethyl, Heptafluorpropyl, Chlorfluormethyl, für Methyl, Ethyl, n-oder i-Propyl, Methoxy, Ethoxy oder Methylthio steht,

$R^2$ für Wasserstoff, Fluor oder Chlor steht,

$R^3$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht,

A für gegebenenfalls ein-bis fünffach durch Chlor substituiertes Alkylen mit 1 bis 8 Kohlenstoffatomen steht und

m für eine Zahl 0, 1 oder 2 steht.

Im einzelnen seien außer den bei den Herstellungsbeispielen genannten Verbindungen die folgenden 5-Fluoracylamino-4-nitro-1-aryl-pyrazole der allgemeinen Formel (I) genannt:

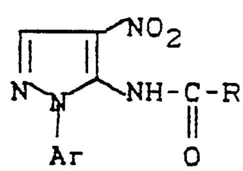

(I)

Tabelle 1

| R | Ar | R | Ar |
|---|---|---|---|
| $-(CF_2)_2-CF_3$ | 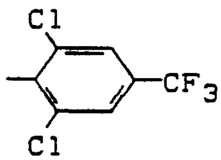 | $-CH_2-CF_3$ | 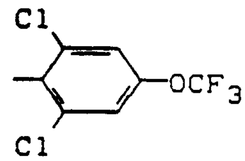 |
| $-(CF_2)_2-CF_3$ | 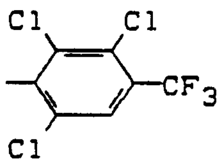 | $-CF_2-CHClF$ | 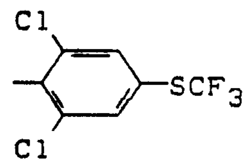 |
| $-CF_2-CF_3$ | 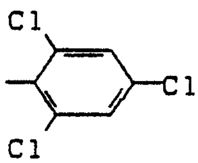 | $-(CF_2)_7-CF_3$ | 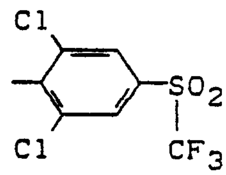 |
| $-(CF_2)_2-CF_3$ | 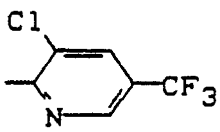 | 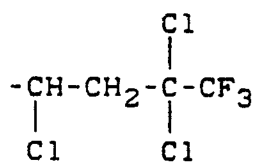 | 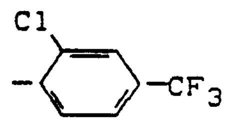 |
| $-CHF-CH_3$ | 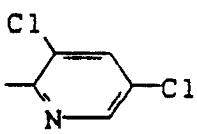 | $-CF_2-CF_3$ | 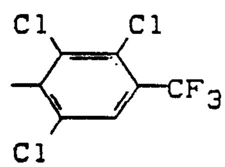 |
| $-CF_2-SCH_3$ | 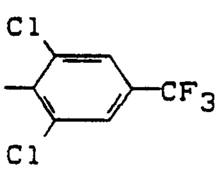 | $-CF_3$ | 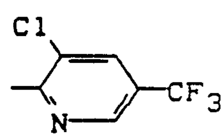 |

Tabelle 1 (Fortsetzung)

| R | Ar | R | Ar |
|---|---|---|---|
| $-\underset{\underset{OCH_3}{\vert}}{C}F-CF_3$ | 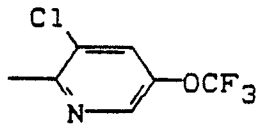 | $-CF_3$ | 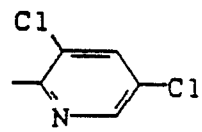 |
| $-CF_3$ | 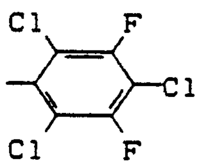 | $-CHF_2$ | 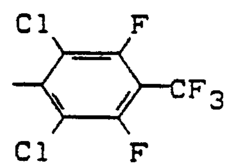 |
| $-CF_3$ | 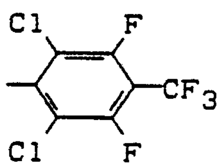 | $-CHF_2$ | 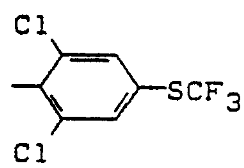 |
| $-CF_3$ | 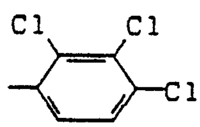 | $-CHF_2$ | 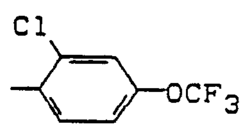 |
| $-CF_3$ | 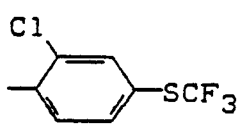 | $-CClF_2$ | 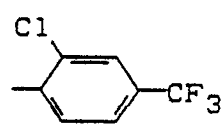 |
| $-CF_3$ | 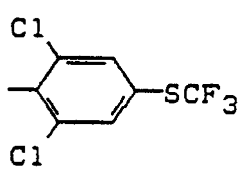 | $-CClF_2$ | 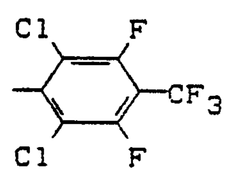 |

<u>Tabelle 1</u> (Fortsetzung)

| R | Ar | R | Ar |
|---|---|---|---|
| $-CF_3$ | 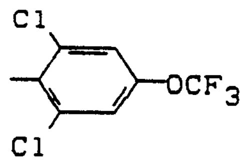 | $-CClF_2$ | 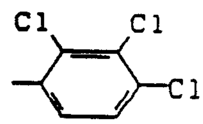 |
| $-CF_3$ | 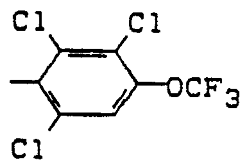 | $-CClF_2$ | 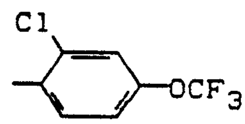 |
| $-CF_3$ | 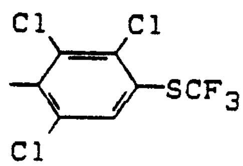 | $-CClF_2$ | 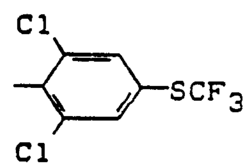 |
| $-CHF_2$ | 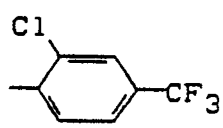 | $-CCl_2F$ | 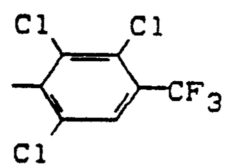 |
| $-CHF_2$ | 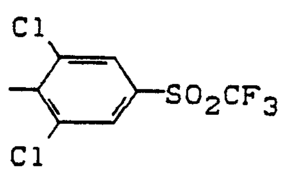 | $-CCl_2F$ | 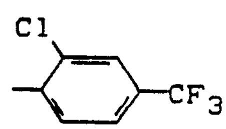 |
| $-CCl_2F$ | 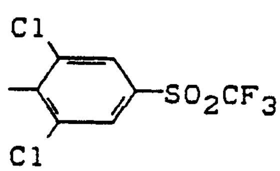 | $-CCl_2F$ | 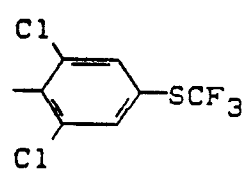 |

Tabelle 1 (Fortsetzung)

| R | Ar | R | Ar |
|---|---|---|---|
| $-CCl_2F$ | 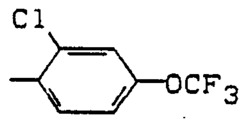 | $-CHF_2$ | 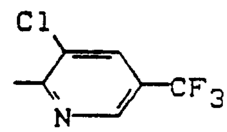 |
| $-CHF_2$ | 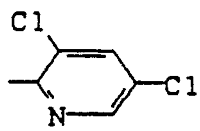 | $-CClF_2$ | 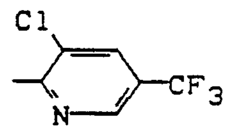 |
| $-CClF_2$ | 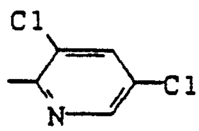 | $-CCl_2F$ | 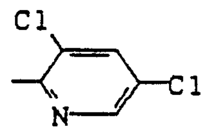 |
| $-CCl_2F$ | 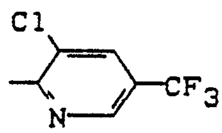 | $-CF_2CF_3$ | 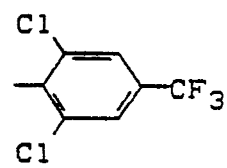 |

Verwendet man beispielsweise 5-Amino-4-nitro-1-(2,6-dichlor-4-trifluormethylphenyl)-pyrazol und Trifluoracetanhydrid als Ausgangsstoffe,so läßt sich der Reaktionsablauf des erfindungsgemäßen Verfahrens - (a) durch das folgende Formelschema darstellen:

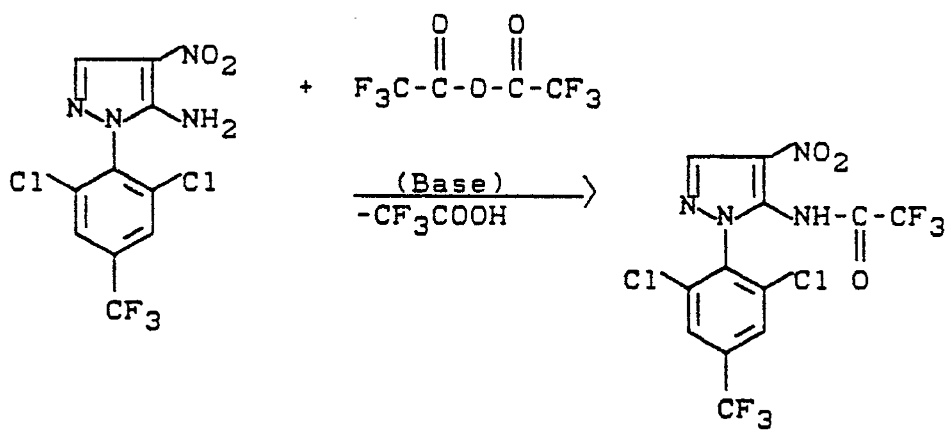


Verwendet man beispielsweise 5-Trifluoracetamido-1-(2,3,6-trichlor-4-trifluormethylphenyl)-pyrazol als Ausgangsverbindung, so läßt sich der Reaktionsablauf deserfindungsgemäßen Verfahren (b) durch das folgende Formelschema darstellen:

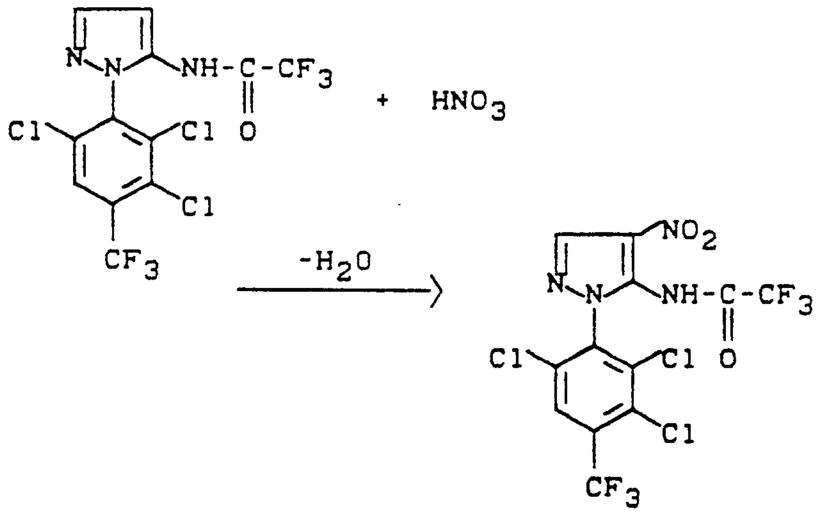


Die zur Durchführung des erfindungsgemäßen Verfahrens (a) als Ausgangsstoffe benötigten 5-Amino-4-nitro-pyrazole sind durch die Formel (II) allgemein definiert. In dieser Formel (II) steht Ar vorzugsweise für diejenigen Reste die bereits im Zusammenhang mit der Beschreibung der erfindungsgemäßen Stoffe der Formel (I) als bevorzugt für diesen Substituenten genannt wurde.

Die 5-Amino-4-nitro-pyrazole der Formel (II) sind bekannt (vergl. DE-OS 34 02 308) oder sind Gegenstand der eigenen vorgängigen Patentanmeldung DE-P 35 20 330 vom 7.6.1985 und erhältlich nach bekannten Verfahren (vergl. DE-OS 34 02 308) beispielsweise, wenn man Arylhydrazine der Formel (V),

$Ar -NH -NH_2$ (V)

in welcher

Ar die oben angegebene Bedeutung hat, mit 2-Halogenacrylnitrilen der Formel (VI),

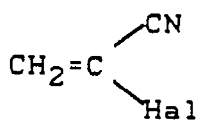

(VI)

in welcher

Hal für Halogen, insbesondere für Chlor oder Brom steht,

entweder zunächst in einer 1. Stufe, gegebenenfalls in Gegenwart eines Verdünnungsmittels wie beispielsweise Eisessig oder Ethanol sowie gegebenenfalls in Gegenwart eines Reaktionshilfsmittels wie beispielsweise Natriumacetat bei Temperaturen zwischen -20°C und +20°C umsetzt zu den Arylhydrazin-Derivaten der Formel (VII),

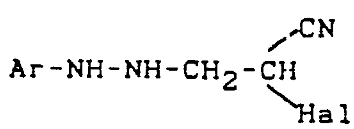

(VII)

in welcher

Ar und Hal die oben angegebene Bedeutung haben,

und diese in einer 2.Stufe, gegebenenfalls in Gegenwart eines Verdünnungsmittels wie beispielsweise Ethylenglykolmonoethylether und gegebenenfalls in Gegenwart eines sauren Katalysators wie beispielsweise Schwefelsäure oder Phosphorsäure bei Temperaturen zwischen +50°C und +150°C cyclisiert,oder direkt in einem Reaktionsschritt, ohne Isolierung der Zwischenstufe der Formel (VII) gegebenenfalls in Gegenwart eines Verdünnungsmittels wie beispielsweise Ethylenglykolmonoethylether oder Ethanol bei Temperaturen zwischen +50°C und +150°C cyclisiert und die so erhältlichen in 4-Stellung unsbustituierten 5-Aminopyrazole der Formel (VIII),

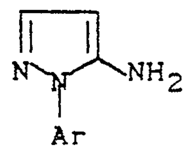

(VIII)

in welcher

Ar die oben angegebene Bedeutung hat, in einer Folgereaktion mit einem Nitrierungsmittel wie beispielsweise Salpetersäure gegebenenfalls in Gegenwart eines Verdünnungsmittels wie beispielsweise Eisessig und gegebenenfalls in Gegenwart eines Reaktionshilfsmittels wie beispielsweise Acetanhydrid bei Temperaturen zwischen -20°C und +50°C nitriert.

Dabei kann es gegebenenfalls von Vorteil sein, vor der Nitrierungsreaktion die Aminogruppe in der 5-Position des Pyrazolringes mit Hilfe üblicher Schutzgruppentechnik, beispielsweise durch Acylierung zu - schützen und die Amino schutzgruppe nach erfolgter Nitrierung ebenfalls in üblicher Art und Weise beispielsweise durch Verseifung mit wäßriger oder alkoholischer Base wieder abzuspalten.

Die Arylhydrazine der Formel (V) sind bekannt (vgl. z.B. US-PS 4 127 575; US-PS 3 609 158; DE-OS 2 558 399; J. Chem. Soc. C, 1971, 167-174) oder können nach bekannten Verfahren in einfacher analoger Weise erhalten werden (vgl. z.B. Houben-Weyl 'Methoden der organischen Chemie' Band X/2, S. 203, Thieme Verlag Stuttgart, 1967), in dem man beispielsweise die bekannten Aniline bzw. Pyridylamine der Formel (IX),

Ar $-NH_2$ (IX)

in welcher

Ar die oben angegebene Bedeutung hat, mit Natriumnitrit in Gegenwart einer Säure wie beispielsweise Schwefelsäure, und dann mit Zinn-II-chlorid ebenfalls in Gegenwart einer Säure wie beispielsweise Salzsäure bei Temperaturen zwischen -20°C und +80°C umsetzt oder wenn man Halogenaromaten der Formel (X)

Ar -Hal' (X)

in welcher

Ar die oben angegebene Bedeutung hat und

Hal' für Halogen, insbesondere für Fluor, Chlor oder Brom steht, mit Hydrazinhydrat gegebenenfalls in Gegenwart eines Verdünnungsmittels wie beispielsweise Pyridin oder Dioxan bei Temperaturen zwischen 0° und 150°C umsetzt.

Die 2-Halogenacrylnitrile der Formel (VI), die Aniline und Pyridylamine der Formel (IX) und die Halogenaromaten der Formel (X) sind allgemein bekannte Verbindungen der organischen Chemie.

Die zur Durchführung des erfindungsgemäßen Verfahrens (a) weiterhin als Ausgangsstoffe benötigten Fluoracyl-Verbindungen sind durch die Formel (III) allgemein definiert. In dieser Formel (III) steht R vorzugsweise für diejenigen Reste, die bereits im Zusammenhang mit der Beschreibung der erfindungsgemäßen Stoffe der Formel (I) als bevorzugt für diesen Substituenten genannt wurden.

E steht vorzugsweise für Halogen, insbesondere Chlor oder Brom oder für einen Rest R-CO-O-, wobei R die oben angegebene Bedeutung hat.

Die Fluoracyl-Verbindungen der Formel (III) sind allgemein bekannte Verbindungen der organischen Chemie.

Die zur Durchführung des erfindungsgemäßen Verfahrens (b) als Ausgangsstoffe benötigten in 4-Stellung unsubstituierten 5-Fluoracylamino-1-aryl-pyrazole sind durch die Formel (IV) allgemein definiert. In dieser Formel (IV) stehen R und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die in 4-Stellung unsubstituierten 5-Fluoracylamino-1-aryl-pyrazole der Formel (IV) sind noch nicht bekannt. Man erhält je jedoch in Analogie zu bekannten Verfahren (vgl. z.B. DE-OS 34 02 308), wenn man 4-unsubstituiert 5-Amino-pyrazole der Formel (VIII),

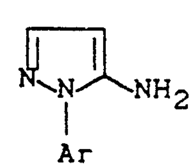

(VIII)

in welcher
Ar die oben angegebene Bedeutung hat, mit Fluoracylverbindungen der Formel (III),

$$R - \underset{\underset{O}{\|}}{C} - E \qquad (III)$$

in welcher
R und E die oben angegebene Bedeutung haben, gegebenenfalls in Gegenwart eines Verdünnungsmittels wie beispielsweise Dichlormethan und gegebenenfalls in Gegenwart eines Säurebindemittels wie beispielsweise Pyridin in Analogie zur Durchführung des erfindungsgemäßen Verfahrens (a) bei Temperaturen zwischen -20°C und +120°C acyliert.

Als Verdünnungsmittel zur Durchführung des erfindungsgemäßen Verfahrens (a) kommen inerte organische Lösungsmittel in Frage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird gegebenenfalls in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxdid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin N,N-Dimethylaminopyridin, Diazabicyclooctan (DABCO), Diazabicyclononen - (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -20°C und +150°C, vorzugsweise bei Temperaturen zwischen 0°C und +120°C.

Zur Durchführung des erfindungsgemäßen Verfahrens (a) setzt man pro Mol an 5-Amino-4-nitro-1-arylpyrazol der Formel (II) im allgemeinen 1,0 bis 5,0 Mol, vorzugsweise 1,0 bis 2,0 Mol an Fluoracyl-Verbindung der Formel (III) und gegebenenfalls 1,0 bis 5,0 Mol, vorzugsweise 1,0 bis 2,0 Mol an Säurebindemittel ein. Die Reaktionsdurchführung, Aufarbeitung und Isolierung der Reaktionsprodukte der Formel (I) erfolgt nach üblichen, allgemein bekannten Verfahren.

Als Verdünnungsmittel zur Durchführung des erfindungsgemäßen Verfahrens (b) kommen alle üblicherweise für derartige. Nitrierungsreaktionen verwendbaren Lösungsmittel in Frage. Vorzugsweise verwendet man die als Reagenzien in Frage kommenden Säuren oder deren Gemische mit Kataly satorsäure, wie beispielsweise Schwefelsäure, Salpetersäure, Acetanhydrid oder Nitriersäure, gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungsmittel in Frage.

Als Katalysatoren oder Reaktionshilfsmittel zur Durchführung des erfindungsgemäßen Verfahrens (b) kommen ebenfalls die für derartige Nitrierungen üblichen Katalysatoren in Frage; vorzugsweise verwendet man saure Katalysatoren, wie beispielsweise Schwefelsäure oder Acetanhydrid.

Die Reaktionstemperaturen können bei der Durchführung des erfindungsgemäßen Verfahrens (b) in einem größeren Bereich variiert werden. Im allgemeinen arbeitet man bei Temperaturen zwischen -50°C und +150°C, vorzugsweise bei Temperaturen zwischen -20°C und +120°C.

Zur Durchführung des erfindungsgemäßen Verfahrens (b) setzt man pro Mol an in 4-Stellung unsubstituiertem 5-Fluoracylamino-1-aryl-pyrazol der Formel (IV) im allgemeinen 1,0 bis 100,0 Mol, vorzugsweise 1,0 bis 50,0 Mol an Salpetersäure und gegebenenfalls 0,1 bis 10 Mol an Katalysator ein. Die Reaktionsdurchführung, Aufarbeitung und Isolierung der Reaktionsprodukte der Formel (I) erfolgt nach üblichen, allgemein bekannten Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono-und dikotyler Unkräuter, insbesondere in monokotylen Kulturen wie Gerste oder Weizen, einsetzen.

Auch die Vorprodukte der Formel (IV) zeigen in entsprechenden Aufwandmengen eine gute herbizide Wirksamkeit.

Darüber hinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare

Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier-und/oder -schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalo stoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Anwendung als Herbizide können die erfindungsgemäßen Wirkstoffe als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Umkrautbekämpfung in Getreide; 4Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischungen mit N,N-Dimethyl-N'-(3-chlor-4-methylphenyl)-harnstoff; N,N-Dimethyl-N'-(4-isopropylphenyl)-harnstoff; 4-Amino-6-t-butyl-3-ethylthio-1,2,4-triazin-5(4H)-on; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; 2-[4-(3,5-Dichlorpyrid-2-yloxy)-phenoxy]-propionsäure-(2-benzyloxyethylester), -(trimethylsilylmethylester) oder -(2,2-diethoxyethylester); Methyl-5-(2,4-dichlorphenoxy)-2-nitrobenzoat; 3,5-Diiod-4-hydroxybenzonitril; 3-Isopropyl-2,1,3-benzothiadiazin-4-on-2,2-dioxid; 2-Chlor-N-{[(4-methoxy-6-methyl-1,3,5-triazin-2-yl)-amino]-carbonyl}-benzolsulfonamid; 4-Ethylamino-2-t-butylamino-6-methylthio-s-triazin; N-Methyl-2-(1,3-benzthiazol-2-yloxy)-acetanilid; N,N-Diisopropyl-S-(2,3,3-trichlorallyl)-thiolcarbamat; N-(1-Ethylpropyl)-3,4-dimethyl-2,6-dinitroanilin; 3,5-Dibrom-4-hydroxy-benzonitril; 2-[4-(2,4-Dichlorphenoxy)-phenoxy]-propionsäure-methylester sind gegebenenfalls von Vorteil.Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Bei der Anwendung als Wachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Ver stäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche 0,01 bis 50 kg, bevorzugten 0,05 bis 10 kg an Wirkstoff.

Für die Anwendungszeit gilt, daß die Anwendung der Wachstumsregulatoren in einem bevorzugt Zeitraum vorgenommen wird, dessen genaue Abgrenzung sich nach den klimatischen und vegetativen Gegebenenheiten richtet.

Die Herstellung und die Verwendung der erfindungsgemäßen Wirkstoffe geht aus den nachfolgenden Beispielen hervor.

Herstellungsbeispiele:

Beispiel 1:

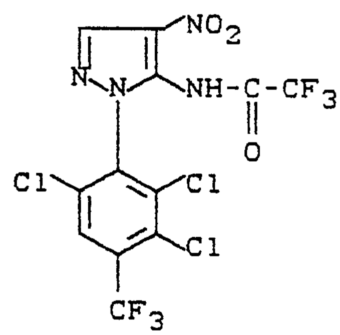

(Verfahren b)

Zu 10 g (0,0235 Mol) 5-Trifluoracetamido-1-(2,3,6-trichlor-4-trifluormethyl-phenyl)-pyrazol in 20 ml Eisessig gibt man bei Raumtemperatur nacheinander 2,4 ml (0.0258 Mol) Acetanhydrid und 1,1 ml (0,025 Mol) 98%ige Salpetersäure. Nach 20-stündigem Rühren wird die Mischung im Vakuum eingeengt, der Rückstand in 100 ml Dichlormethan aufgenommen und mit 400 ml 5%iger wäßriger Natriumcarbonatlösung extrahiert. Die wässrige Phase wird angesäuert und mit 200 ml Dichlormethan extrahiert. Die vereinigten organischen Phasen werden über Natriumsulfat getrocknet und im Vakuum vom Lösungsmittel befreit. Man erhält 8,6 g (77,6 % der Theorie) an 4-Nitro-5-trifluoracetamido-1-(2,3,6-trichlor-4-trifluormethyl-phenyl)-pyrazol vom Schmelzpunkt 136-137°C.

Beispiel 2:

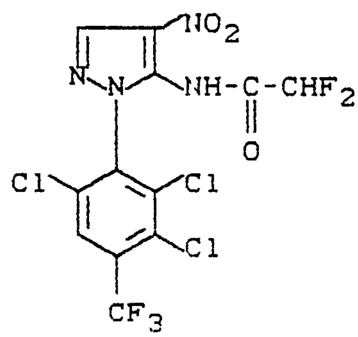

(Verfahren b)

Zu 5,4 g (0,0132 Mol) 5-Difluoracetamido-1-(2,3,6-trichlor-4-trifluormethyl-phenyl)-pyrazol in 30 ml Eisessig gibt man bei Raumtemperatur nacheinander 1,4 ml (0,0145 Mol) Acetanhydrid und 0,6 ml (0,0139 Mol) 98%ige Salpetersäure. Nach 20-stündigem Rühren gießt man das Reaktionsgemisch in 500 ml Wasser, saugt den kristallinen Niederschlag ab und trocknet ihn. Man erhält 5,4 g (91 % der Theorie) an 5-Difluoracetamido-4-nitro-1-(2,3,6-trichlor-4-trifluormethyl-phenyl)-pyrazol vom Schmelzpunkt 146-151 °C.

In entsprechender Weise und gemäß den allgemeinen Angaben zur Herstellung erhält man die folgenden 5-Fluoracylamino-4-nitro-1-aryl-pyrazole der allgemeinen Formel (I):

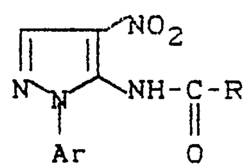

(I)

Tabelle 2

| Bsp. Nr. | R | Ar | Schmelzpunkt (°C) |
|---|---|---|---|
| 3 | $-CF_3$ | 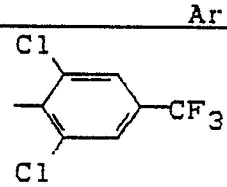 | 145 |
| 4 | $-CF_3$ | 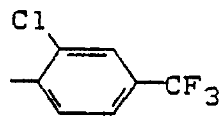 | 98-101 |
| 5 | $-CF_3$ | 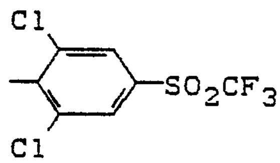 | 166-168 |
| 6 | $-CCl_2F$ | 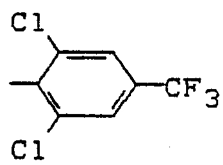 | 128-137 |
| 7 | $-CHF_2$ | 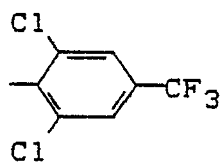 | 93-108 |
| 8 | $-CF_3$ | 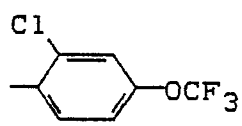 | 59-80 |
| 9 | $-CClF_2$ | 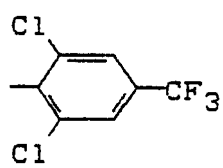 | 116-119 |

Tabelle 2 (Fortsetzung)

| Bsp. Nr. | R | Ar | Schmelzpunkt (°C) |
|---|---|---|---|
| 10 | $-CClF_2$ | 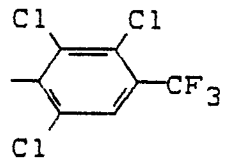 | 105-115 |
| 11 | $-CClF_2$ | 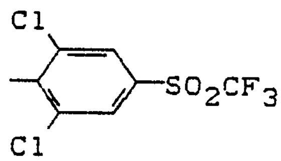 | 171-173 |
| 12 | $-CF_3$ | 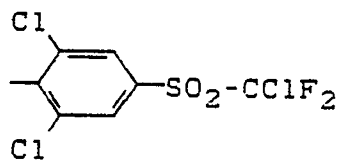 | 168-170 |
| 13 | $-CF_3$ | 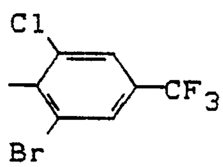 | 138-143 |
| 14 | $-CF_3$ | 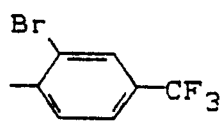 | |
| 15 | $-CF_3$ | 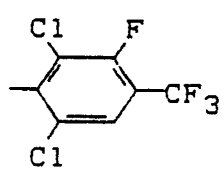 | 139-145 |

Herstellung der Ausgangsverbindungen:

Beispiel IV-1:

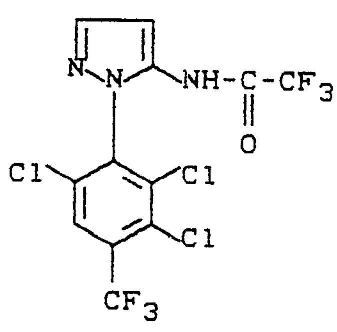

Zu 12 g (0,0363 Mol) 5-Amino-1-(2,3,6-trichlor-4-trifluormethyl-phenyl)-pyrazol in 65 ml Dichlormethan gibt man unter Rühren bei 0°C bis 5°C nacheinander 3,1 ml (0,039 Mol) wasserfreies Pyridin und 5,5 ml - (0,038 Mol) Trifluoracetanhydrid und rührt 6 Stunden bei Raumtemperatur. Zur Aufarbeitung setzt man 50 ml Dichlormethan zu, wäscht nacheinander mit verdünnter Salzsäure, wäßriger Natriumhydrogencarbonat- und Natriumchloridlösung, trocknet die organische Phase über Magnesiumsulfat und entfernt das Lösungsmittel im Vakuum. Man erhält 14,6 g (94,3 % der Theorie) an 5-Trifluoracetamido-1-(2,3,6-trichlor-4-trifluormethyl-phenyl)-pyrazol vom Schmelzpunkt 140-145°C.

In entsprechender Weise und gemäß den allgemeinen Angaben zur Herstellung erhält man die folgenden in 4-Stellung unsubstituierten 5-Fluoracylamino-1-aryl-pyrazole der allgemeinen Formel (IV):

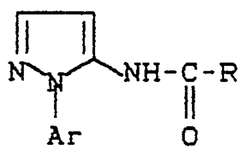

(IV)

Tabelle 3

| Bsp. Nr. | R | Ar | Schmelzpunkt (°C) |
|---|---|---|---|
| IV-2 | $-CHF_2$ | 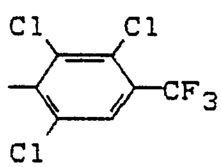 | 120-125 |
| IV-3 | $-CF_3$ | 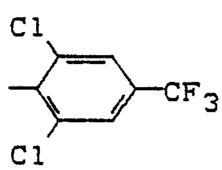 | 152-157 |
| IV-4 | $-CF_3$ | 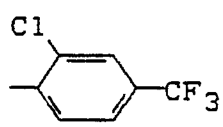 | 150-153 |
| IV-5 | $-CF_3$ | 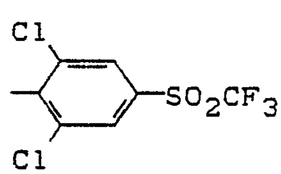 | 162-164 |
| IV-6 | $-CCl_2F$ | 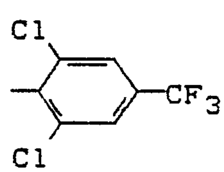 | 151-164 |
| IV-7 | $-CHF_2$ | 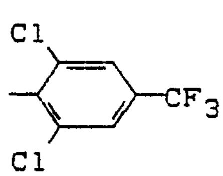 | 121-132 |

<u>Tabelle 3</u> (Fortsetzung)

| Bsp. Nr. | R | Ar | Schmelzpunkt (°C) |
|---|---|---|---|
| IV-8 | $-CF_3$ | 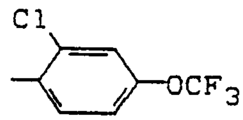 | 112-115 |
| IV-9 | $-CClF_2$ | 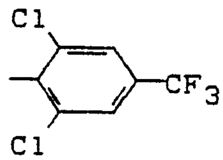 | 155-158 |
| IV-10 | $-CClF_2$ | 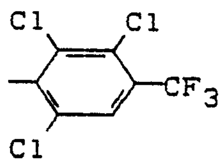 | 160-169 |
| IV-11 | $-CClF_2$ | 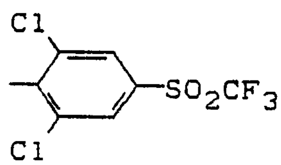 | 139-144 |
| IV-12 | $-CF_3$ | 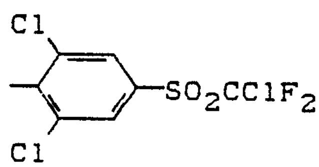 | 151-154 |
| IV-13 | $-CF_3$ | 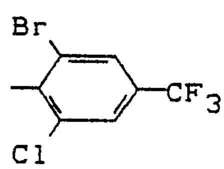 | 170 |
| IV-14 | $-CF_3$ | 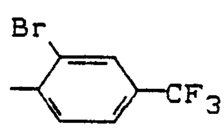 | |
| IV-15 | $-CF_3$ | 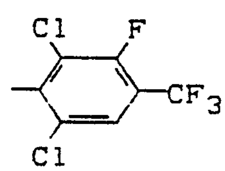 | 148-151 |

Anwendungsbeispiele:

In den folgenden Anwendungsbeispielen wurde die nachstehend aufgeführte Verbindung als Vergleichsubstanz eingesetzt:

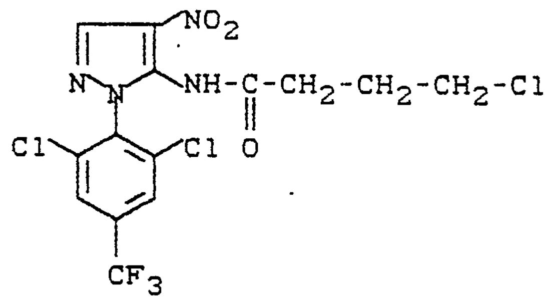

(A)

5-(ω-Chlorbutyramido)-4-nitro-1-(2,6-dichlor-4-trifluormethyl-phenyl)-pyrazol
(bekannt aus DE-OS 34 02 308)

Beispiel A

Pre-emergence-Test
Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit gegenüber der Vergleichssubstanz (A) zeigt in diesem Text z.B. die Verbindung gemäß dem Herstellungsbeispiel: 1.

Beispiel B

Post-emergence-Test
Lösungsmittel: 5 Gewichtsteil Aceton
Emulgator: 1 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber der Vergleichssubstanz (A) zeigt in diesem Test z.B. die Verbindung gemäß dem Herstellungsbeispiel: 1.

Beispiel C

Entlaubung und Austrocknung der Blätter bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall

+ leichtes Austrocknen der Blätter, geringer Blattfall

+ + starkes Austrocknen der Blätter, starker Blattfall

+ + + sehr starkes Austrocknen der Blätter, sehr starker Blattfall

In diesem Test zeigen beispielsweise die Verbindungen gemäß den Herstellungsbeispielen 1, 3, 4, 5, 6 und 7 eine deutliche Überlegenheit im Vergleich zur unbehandelten Kontrolle.

**Ansprüche**

1. 5-Fluoracylamino-4-nitro-1-aryl-pyrazole der allgemeinen Formel (I),

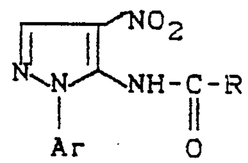

(I)

in welcher

R für durch Fluor substituiertes Alkyl steht und

Ar für jeweils gegebenenfalls substituiertes Phenyl oder Pyridyl steht.

2. 5-Fluoracylamino-4-nitro-1-aryl-pyrazole der Formel (I) gemäß Anspruch 1, in welcher

R für einen Rest 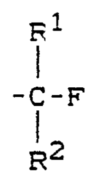 oder für einen Rest $-A-CF_3$ steht und

Ar für gegebenenfalls einfach oder mehrfach, gleich oder verschieden substituiertes Phenyl oder für jeweils gegebenenfalls einfach oder mehrfach, gleich oder verschieden substituiertes 2-Pyridyl, 3-Pyridyl oder 4-Pyridyl steht, wobei als Substituenten jeweils in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_m-R^3$,

wobei

$R^1$ für Wasserstoff, Fluor, Chlor, geradkettiges oder verzweigtes Halogenalkyl mit 1 bis 4 Kohlenstoffatomen und 1 bis 9 gleichen oder verschiedenen Halogenatomen, oder für jeweils geradkettiges oder verzweigtes Alkyl, Alkoxy oder Alkylthio mit jeweils 1 bis 4 Kohlenstoffatomen steht,

$R^2$ für Wasserstoff, Fluor oder Chlor steht,

$R^3$ für Amino sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleicher oder verschiedenen Halogenatomen steht,
A für gegebenenfalls einfach oder mehrfach durch Chlor substituiertes Alkylen mit 1 bis 12 Kohlenstoffatomen steht und
m für eine Zahl 0, 1 oder 2 steht.

3. 5-Fluoracylamino-4-nitro-1-aryl-pyrazole der Formel (I) gemäß Anspruch 1, in welcher

R für einen Rest 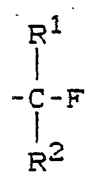 oder für einen Rest $-A-CF_3$ steht

und
Ar für gegebenenfalls ein-bis fünffach, gleich oder verschieden substituiertes Phenyl oder für jeweils gegebenenfalls ein-bis vierfach, gleich oder verschieden substituiertes 2-Pyridyl, 3-Pyridyl oder 4-Pyridyl steht, wobei als Substituenten jeweils in Frage kommen: Cyano, Nitro, Fluor, Chlor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^3$, wobei
$R^1$ für Wasserstoff, Fluor, Chlor, Trifluormethyl, Difluormethyl, Fluormethyl, Dichlorfluormethyl, Difluorchlormethyl, Chlormethyl, Dichlormethyl, Trichlormethyl, Pentafluormethyl, Heptafluorpropyl, Chlorfluormethyl, für Methyl, Ethyl, n-oder i-Propyl, Methoxy, Ethoxy oder Methylthio steht,
$R^2$ für Wasserstoff, Fluor oder Chlor steht,
$R^3$ für Amino, Methylamino, Ethylamino, Dimethylamino, Diethylamino, Fluordichlormethyl, Difluormethyl, Tetrafluormethyl, Trifluorchlorethyl, Trifluormethyl, Methyl oder Ethyl steht,
A für gegebenenfalls ein-bis fünffach durch Chlor substituiertes Alkylen mit 1 bis 8 Kohlenstoffatomen steht und
m für eine Zahl 0, 1 oder 2 steht.

4. Verfahren zur Herstellung von 5-Fluoracylamino-4-nitro-1-aryl-pyrazolen der Formel (I),

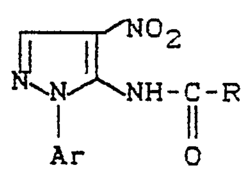 (I)

in welcher
R für durch Fluor substituiertes Alkyl steht und
Ar für jeweils gegebenenfalls substituiertes Phenyl oder Pyridyl steht,
dadurch gekennzeichnet, daß man
(a) 5-Amino-4-nitro-1-aryl-pyrazole der Formel (II),

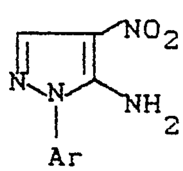 (II)

in welcher
Ar die oben angegebene Bedeutung hat, mit Fluoracyl-Verbindungen der Formel (III),

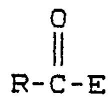

(III)

in welcher
R die oben angegebene Bedeutung hat und
E für eine elektronenanziehende Abgangsgruppe steht,
gegebenenfalls in Gegenwart eines Verdünnungsmittels und gegebenenfalls in Gegenwart eines Säurebindemittels umsetzt, oder daß man
(b) in 4-Stellung unsubstituierte 5-Fluoracylamino-1-aryl-pyrazole der Formel (IV),

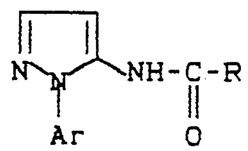

(IV)

in welcher
R die oben angegebene Bedeutung hat, mit Salpetersäure gegebenenfalls in Gegenwart eines Verdünnungsmittels und gegebenenfalls in Gegenwart eines Katalysators umsetzt.

5. Herbizide und pflanzenwuchsregulierende Mittel, gekennzeichnet durch einen Gehalt an mindestens einem 5-Fluoracylamino-4-nitro-1-aryl-pyrazol der Formel (I) gemäß den Ansprüchen 1 bis 4.

6. Verfahren zur Bekämpfung von Unkräutern, dadurch gekennzeichnet, daß man 5-Fluoracylamino-4-nitro-1-aryl-pyrazole der Formel (I) gemäß den Ansprüchen 1 bis 4 auf die Unkräuter und/oder ihren Lebensraum einwirken läßt.

7. Verwendung von 5-Fluoracylamino-4-nitro-1-aryl-pyrazolen der Formel (I) gemäß den Ansprüchen 1 bis 4, zur Bekämpfung von Unkräutern und/oder als Pflanzenwachstumsregulatoren.

8. Verfahren zur Herstellung von herbiziden und/oder pflanzenwuchsregulierenden Mitteln, dadurch gekennzeichnet, daß man 5-Fluoracylamino-4-nitro-1-aryl-pyrazolen der Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung

EP 86 11 6245

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | DE-A-3 402 308 (BAYER AG) * Seite 11, Zeile 1 - Seite 13, Zeile 6, besonders Seite 11, Zeile 3, Seite 12, Zeilen 21, 24; Seite 34, Zeile 19 - Seite 36, Zeile 25; Ansprüche 2-4 * | 1,4-8 | C 07 D 231/40<br>C 07 D 401/04<br>A 01 N 43/56 |
| A | FR-A-2 503 706 (SHOWA DENKO K.K.) * Tabelle I, Verbindungen 5, 34; Ansprüche 1-3, 7, 12, 13 * | 1,4,5 | |
| E,A D | DE-A-3 520 330 (BAYER AG) * Seite 44, Zeile 23 - Seite 45, Zeile 36, Seite 46, Zeile 34 - Seite 48, Zeile 14, Seite 60, Zeile 1- Seite 63, Zeile 15, besonders Seite 60, Zeile 10, Seite 61, Zeilen 25, 26, 29; Ansprüche 5-8 * | 1,4-8 | |

RECHERCHIERTE SACHGEBIETE (Int. Cl.4)

C 07 D 231/00
C 07 D 401/00
A 01 N 43/00

Der vorliegende Recherchenbericht wurde für alle Patentansprüche erstellt.

| Recherchenort | Abschlußdatum der Recherche | Prüfer |
|---|---|---|
| BERLIN | 17-02-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82